# EUROPEAN PATENT SPECIFICATION

(11) **EP 0 174 981 B1**
(45) Date of publication and mention of the grant of the patent: **08.02.1995**
(21) Application number: 85901682.6
(22) Date of filing: 01.03.1985
(51) Int. Cl.: A61K 38/00, A61K 33/32, C07C 229/00

(54) **METHOD OF TREATING DEPRESSION IN VERTEBRATES**
BEHANDLUNGSVERFAHREN FÜR DEPRESSIONEN BEI WIRBELTIEREN
PROCEDE DE TRAITEMENT DE LA DEPRESSION CHEZ LES VERTEBRES

(30) Priority: 01.03.1984 US 585291
(43) Date of publication of application: 26.03.1986
(73) Proprietor: ERK, Vernon, New York, NY 10170 (US)
(72) Inventor: ERK, Vernon, New York, NY 10170 (US)
(74) Representative: Perry, Robert Edward
(86) International application number: US8500331
(87) International publication number: WO8503874

(56) References cited:
- WO-A-82/00250
- LU-A- 67 165
- LU-A- 67 169
- US-A- 3 553 258
- US-A- 3 873 296
- US-A- 4 167 564
- US-A- 4 218 474
- US-A- 4 435 424
- CHEMICAL ABSTRACTS, vol. 91, 1979, Columbus, OH (US); CHANDRA, no. 33697r#
- CHEMICAL ABSTRACTS, vol. 90, 1979, Columbus, OH (US); SHUKLA, no. 198507v#
- CHEMICAL ABSTRACTS, vol. 99, 1983, Columbus, OH (US); CHANDRA, no. 100473u#
- CHEMICAL ABSTRACTS, vol. 98, 1983, Columbus, OH (US); KALIMAN, no. 195890u#

## Description

This invention relates to the treatment of depression in vertebrates.

Chem. Abs. 99(1983) 100473u discloses that the hyperactivation produced by administration of manganese is associated with increased levels of dopamine and norepinephrine.

WO-A-8200250 discloses that depression in human patients can be alleviated by the administration of tyrosine and/or phenylalanine, in order to regulate blood plasma levels of tyrosine or phenylalanine and thereby to form corresponding amounts of catecholamines released in synapses.

The present invention, refers to the use of (a) an amino-acid selected from L- or D-phenylalanine, L- or D-tyrosine and α-keto and α-hydroxy analogues thereof, acetyl-L- or D-tyrosine, or a dipeptide or tripeptide form thereof, or a pharmaceutically-acceptable acid addition salt thereof, and (b) a manganese compound, for the preparation of a medicament for use in treating depression in vertebrates.

Because of the cumulative nature of the medication, treatment should begin with small amounts and work up to maximum doses. Then, almost as quickly over a period of days, it may be necessary to decrease the dosage. The manganese may be considered to modulate the phenylalanine or other anti-depressant amino-acid in its action as a precursor for catecholamines.

Depression is best evaluated by the clinician, in initial treatment. This is dictated by changes in dosage that sometime occur very quickly. The patient should be taken off all drugs when beginning treatment, since it is important that the clinician has an accurate picture of the patient, without masking by the effect of other drugs.

The following Example illustrates the invention.

### Example 1

### Patient B.E. midlife

History of recurrent attacks of depression. Periods of stress have contributed to recurrent episodes at rather long intervals of time.

Treatment periods: Circumstances prevented daily visits during one episode of depression. Another episode medication could be provided daily.

Treatment: Evaluation of emotional status. Dietary management for stabilization of blood sugar. Placed on combination of phenylalanine and manganese (doses calculated in mg of manganese in manganese gluconate). Initial therapy with 3 to 4 mg of manganese. Phenylalanine begun with about ten to twenty mg. These were increased to about 4 to 8 mg of manganese and then decreased when this amount was no longer needed. The phenylalanine was increased to 25 to 50 mg. Its dosage was then given at less frequent intervals and in decreasing amounts.

Treatment Period Interval: Initially it varied from daily to every two or three days. After ten to fourteen days, every three or four days for a week or two more. This changed quickly to once a week, then every ten to fourteen days and then every three to four weeks. Dietary program became sufficient with intervals at one to two months.

Objective of Treatment: To restore emotional tone to normal range.

Clinical Response: Normal affect and emotional pattern with full work schedule within two to three weeks. Full normal pattern of response delayed to about six to eight weeks. Supportive treatment at intervals.

Second episode: Very little time for treatment. Followed as above about one week with same degree of improvement. Then, given about 8 to 12 mg of manganese as gluconate and 50 mg or more mg of phenylalanine at weekly or longer intervals. Rapid improvement. Continued full schedule of work.

Ratios of medication: B.W. 75 kg. Mn ranged from 0.040 mg to 0.106 mg per kg; phenylalanine from 0.133 to 0.667 mg/kg
Phenylalanine was given on an average of three to five times as often as manganese.

## Claims

1. Use of (a) an amino-acid selected from L- or D-phenylalanine, L- or D-tyrosine and α-keto and α-hydroxy analogues thereof, acetyl-L- or D-tyrosine, or a dipeptide or tripeptide form thereof, or a pharmaceutically-acceptable acid addition salt thereof, and (b) a manganese compound, for the preparation of a medicament for use in treating depression in vertebrates.

2. Use of an amino-acid as defined in claim 1 or a manganese compound, for the manufacture of a medicament for use in treating depression in vertebrates to which has previously been administered the manganese compound or the amino-acid, respectively.

3. Use according to claim 1 or claim 2, wherein the manganese compound is manganese gluconate.

## Patentansprüche

1. Verwendung von (a) einer Aminosäure, ausgewählt aus L- oder D-Phenylalanin, L- oder D-Tyrosin und α-Keto- und α-Hydroxyanalogen davon, Acetyl-L- oder -D-tyrosin oder einer Dipeptid- oder Tripeptid-Form davon oder einem pharmazeutisch annehmbaren Säureadditionssalz davon und (b) einer Manganverbindung für die Herstellung eines Medikaments zur Verwendung bei der Behandlung der Depression bei Vertebraten.

2. Verwendung einer Aminosäure, wie in Anspruch 1 definiert, oder einer Manganverbindung für die Herstellung eines Medikaments zur Verwendung bei der Behandlung der Depression bei Vertebraten, denen vorher die Manganverbindung bzw. die Aminosäure verabreicht worden ist.

3. Verwendung nach Anspruch 1 oder Anspruch 2, worin die Manganverbindung Mangangluconat ist.

## Revendications

1. Utilisation (a) d'un acide aminé choisi parmi la L-phénylalanine ou la D-phénylalanine, la L-tyrosine ou la D-tyrosine ou un de leurs analogues de type α-céto et α-hydroxy, l'acétyl-L-tyrosine ou l'acétyl-D-tyrosine ou leur forme dipeptidique ou tripeptidique, ou de leur sel d'addition d'acide pharmaceutiquement acceptable et (b) d'un composé manganèse, pour la préparation d'un médicament à utiliser dans le traitement d'une dépression chez les vertébrés.

2. Utilisation d'un acide aminé tel que défini dans la revendication 1 ou d'un composé manganèse pour la production d'un médicament à utiliser dans le traitement d'une dépression chez les vertébrés auxquels le composé manganèse ou l'acide aminé, respectivement, a été au préalable administré.

3. Utilisation selon la revendication 1 ou la revendication 2, pour laquelle le composé manganèse est du gluconate de manganèse.
